# EUROPEAN PATENT SPECIFICATION

(11) **EP 2 771 013 B1**
(45) Date of publication and mention of the grant of the patent: **13.07.2016**
(21) Application number: 12784709.3
(22) Date of filing: 28.09.2012
(51) Int. Cl.: A61K 31/522, A61K 31/728, A61K 9/00, A61K 47/36

(54) **TOPICAL PHARMACEUTICAL COMPOSITIONS COMPRISING ACYCLOVIR AND HYALURONIC ACID**
TOPISCHE PHARMAZEUTISCHE ZUSAMMENSETZUNGEN MIT ACYCLOVIR UND HYALURONSÄURE
COMPOSITIONS PHARMACEUTIQUES TOPIQUES COMPRENANT DE L'ACYCLOVIR ET DE L'ACIDE HYALURONIQUE

(30) Priority: 28.09.2011 IT MI20111747
(43) Date of publication of application: 03.09.2014
(73) Proprietor: Fidia Farmaceutici S.p.A., 35031 Abano Terme (PD) (IT)
(72) Inventor: CALLEGARO, Lanfranco, I-35031 Abano Terme (PD) (IT); GENNARI, Giovanni, I-35031 Abano Terme (PD) (IT)
(74) Representative: Bertuccio, Silvia
(86) International application number: PCT/IB2012/055181
(87) International publication number: WO 2013/046161

(56) References cited:
- WO-A1-98/18472
- WO-A1-2009/115510
- WO-A2-2009/031006
- GIUSEPPINA SANDRI ET AL: "Mucoadhesive and penetration enhancement properties of three grades of hyaluronic acid using porcine buccal and vaginal tissue, Caco-2 cell lines, and rat jejunum", JOURNAL OF PHARMACY AND PHARMACOLOGY, LONDON; GB, vol. 56, no. 9, 1 September 2004 (2004-09-01), pages 1083-1090, XP002667561, ISSN: 0022-3573, DOI: 10.1211/0022357044085 [retrieved on 2010-02-18]
- TRABUCCHI E ET AL: "LOW MOLECULAR WEIGHT HYALURONIC ACID PREVENTS OXYGEN FREE RADICAL DAMAGE TO GRANULATION TISSUE DURING WOUND HEALING", INTERNATIONAL JOURNAL OF TISSUE REACTIONS, BIOSCIENCE EDIPRINT, GENEVA, CH, vol. 24, no. 2, 1 January 2002 (2002-01-01), pages 65-71, XP009072451, ISSN: 0250-0868

## Description

### Field of invention

The present invention relates to topical pharmaceutical compositions in the form of sticks, O/W emulsions or vaginal pessaries containing acyclovir and a hyaluronic acid salt, and their use in the treatment of pain associated with *Herpes simplex labialis* and *genitalis* infections.

### Prior art

*H. simplex* infections are very frequent, and though not usually dangerous to the patient's overall state of health, are generally poorly tolerated. They take the form of eruptions consisting of a number of blisters that contain the live virus during the clinical stage. These blisters are painful, particularly at the start of the infection, and very often burst, forming scabs which are also painful. There are two subtypes of the *Herpes simplex* virus (HSV-1 and HSV-2), each of which tends to infect different parts of the human body. In particular, HSV-1 appears on the lips (*H. simplex labialis*), whereas subtype HSV-2 infects the mucosa of the vulva and penis (*H. simplex genitalis*). Primary HSV-2 lesions, which are more extensive and serious, can expand towards the medial surface of the thighs, the perianal area, the vagina, the anus and the rectal mucosa. The drugs known for antiviral activity which are used in these situations include acyclovir. Said medicament exercises an intense antiviral activity; it inhibits viral replication, preventing or greatly slowing the progress of the infection, and is used both topically and systemically. For example, WO98/18472 discloses pharmaceutical compositions for the treatment of *H. simplex labialis;* said compositions, in stick or roll-on form, consist of a mixture of waxy substances and contain acyclovir, either alone or combined with vitamin A.

Hyaluronic acid (HA) is a heteropolysaccharide composed of alternate residues of D-glucuronic acid and N-acetyl-D-glucosamine.

It is a linear-chain polymer with a molecular weight ranging between 50,000 and 13 x 10⁶ Da, depending on the source from which it is obtained and the preparation methods used.

It is found in nature in pericellular gels, in the ground substance of the connective tissue of vertebrates (of which it is one of the main components), and in synovial (joint) fluid, vitreous humour and the umbilical cord.

HA plays an important role in biological organisms, especially as a mechanical support for the cells of many tissues such as skin, tendons, muscles and cartilage.

It is also known that HA, through its CD44 membrane receptor, modulates many different processes relating to cell physiology and biology such as cell proliferation, migration, differentiation and angiogenesis, and also performs other functions such as hydrating the tissues and lubricating the joints.

It has been demonstrated that HA plays a crucial role in the tissue repair process, firstly from the structural standpoint (by organising the extracellular matrix and regulating its hydration), and secondly as a substance that stimulates a wide range of processes in which it acts directly and/or indirectly (clot formation, phagocyte activity, fibroblast proliferation, neovascularisation, re-epithelialisation, etc.) (Weigel P. et al., J Theoretical Biol, 1986:219-234; Abatangelo G. et al., J Surg Res, 1983, 35:410-416; Goa K. et al., Drugs, 1994, 47:536-566).

These well-recognised properties have long been exploited to prepare dressings used in the care of wounds, ulcers and skin lesions of various origins, and in all skin lesions that benefit from accelerated re-epithelialisation. For example, EP197718 discloses medicaments for topical use based on hyaluronic acid and pharmacologically active substances; the hyaluronic acid used has a molecular weight of 50-100 kDa or 500-730 kDa.

WO2009/115510 discloses compositions for treating herpes virus infection comprising an acyclic guanosine analogue selected from acyclovir, penciclovir and omaciclovir in an oil in water or water in oil carrier comprising propylene glycol and isopropyl alkanoic acid ester. However, there is still a need to identify topical pharmaceutical compositions for the prevention and/or treatment of *Herpes simplex labialis* or *genitalis,* which act on the virus as such, deactivating it, and promote the re-epithelialisation of the mucosa after the blister bursts and the scab appears, and above all to act effectively on the pain symptoms, reducing or eliminating them.

### Summary of the invention

The present invention relates to topical painkilling compositions containing acyclovir at a concentration of between 2 and 8% w/w and hyaluronic acid in the form of sodium salt with a molecular weight of between 160 and 200 kDa at a concentration of between 0.1 and 1% w/w for use in the prevention and/or treatment of the pain associated with *Herpes simplex labialis* or *genitalis* infections.

### List of figures

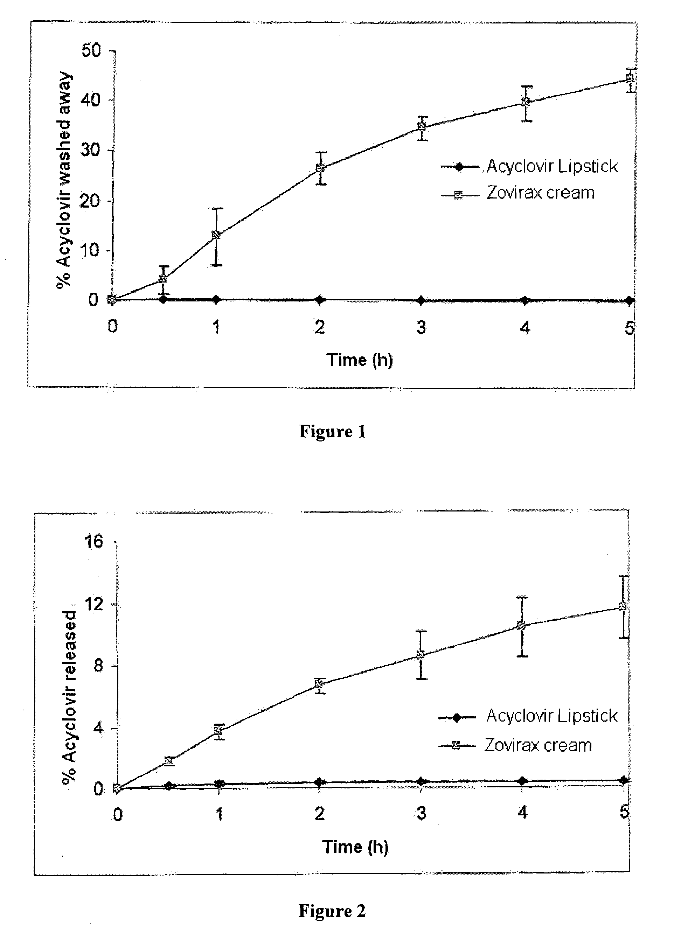

Figure 1 shows the percentage profiles of the active ingredient washed away by the stick compositions according to the present invention containing 5% w/w acyclovir and by Zovirax® cream containing 5% w/w acyclovir, obtained from simultaneous leachability and release measurements (mean values ± SD; n=3).
Figure 2 shows the percentage profiles of the active ingredient released by the stick compositions according to the present invention containing 5% w/w acyclovir and by Zovirax® cream containing 5% w/w acyclovir, obtained from simultaneous leachability and release measurements (mean values ± SD; n=3).

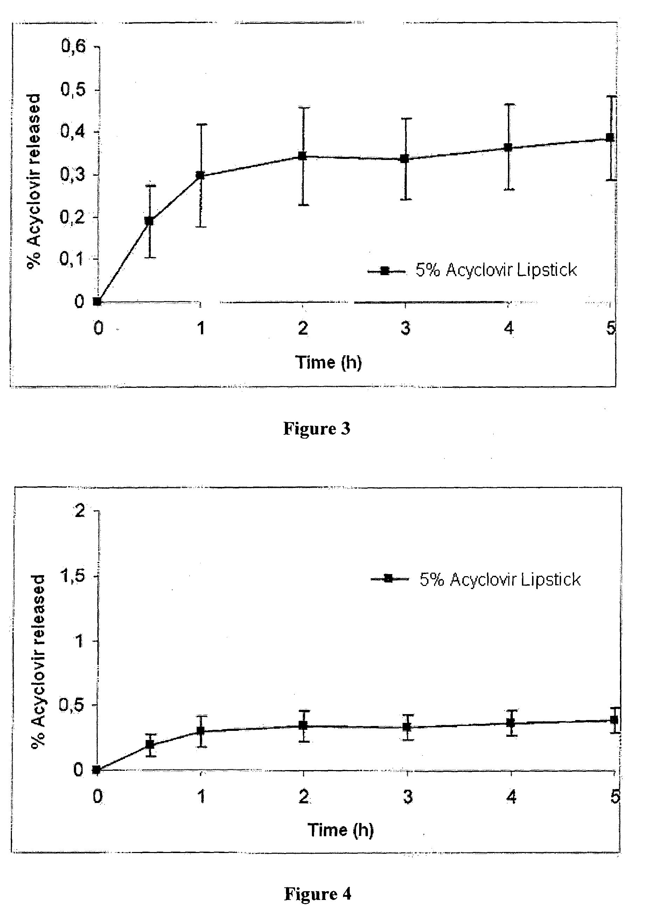

Figure 3 and Figure 4 show the percentage profiles of the active ingredient released by the stick compositions according to the present invention containing 5% w/w acyclovir.

### Detailed description of the invention

The present invention relates to topical painkilling pharmaceutical compositions which may be in the form of sticks, O/W emulsions or vaginal pessaries containing acyclovir and hyaluronic acid in the form of sodium salt (sodium hyaluronate).

Acyclovir is present in concentrations ranging between 2 and 8%, preferably between 3 and 6%, and even more preferably amounting to 5% by weight of the total pharmaceutical composition, depending on the pharmaceutical form selected.

Acyclovir, used according to the present invention, can be micronised (acyclovir M) and have the following particle-size distribution:
- d60 NMT 5 microns
- d 100 NMT 20 microns.

It is known that acyclovir can take different polymorphic forms, namely:
- water-solvated form (1 mole) which loses the solvation solvent between 50 and 150°C; after desolvation the product is transformed into the form that breaks down at 230-250°C;
- anhydrous form which melts with breakdown at 230-250°C;
- unstable anhydrous form which melts at 171°C and is transformed into the high-melting form under heating.

According to a preferred aspect, the polymorphic form of acyclovir is preferably the water-solvated form.

The pharmaceutical compositions contain hyaluronic acid in the form of sodium salt. Sodium hyaluronate can be present in concentrations ranging between 0.1 and 1%, preferably 0.2% by weight of the total pharmaceutical composition, and has a molecular weight of between 160 and 200 kDa, hereinafter indicated for the sake of brevity as mean MW 200 kDa. References to molecular weight (MW) according to the present invention refer to the weight-average molecular weight, calculated by the "intrinsic viscosity" method (Terbojevich et al., Carbohydr Res, 1986, 363-377).

The hyaluronic acid used in the present invention can derive from any source; for example, it can be obtained by extraction from rooster combs (EP 138572 B1), by fermentation (from *Streptococcus equi* or *zooepidemicus*), or by biosynthesis (from *Bacillus*), as known to one skilled in the art.

According to a preferred aspect of the invention, the topical painkilling pharmaceutical compositions according to the present invention consist of:
- a water-in-oil (W/O) emulsion in the form of a solid stick containing acyclovir at a concentration of between 2 and 8%, preferably between 3 and 6%, and even more preferably 5% by weight, and sodium hyaluronate with an average MW of between 160 and 200 kDa, at a concentration of between 0.1 and 1%, preferably 0.2% by weight, for use in the prevention and/or treatment of the pain associated with *H. simplex labialis* infections;
- an oil-in-water emulsion (O/W emulsion) in cream form containing acyclovir at a concentration of between 2 and 8%, preferably between 3 and 6%, and even more preferably 5% by weight, and sodium hyaluronate with an average MW of between 160 and 200 kDa, at a concentration of between 0.1 and 1%, preferably 0.2% by weight, for use in the prevention and/or treatment of the pain associated with *H. simplex labialis* or *genitalis* infections;
- vaginal pessaries containing acyclovir at a concentration of between 3 and 6%, preferably 5% by weight, and sodium hyaluronate with an average MW of between 160 and 200 kDa, at a concentration of between 0.1 and 1%, preferably 0.2% by weight, for use in the prevention and/or treatment of the pain associated with *H. simplex genitalis* infections.

At the stage of preparation of the solid stick (lipstick), the base of the lipophilic phase of the emulsion can consist of a lipophilic solvent (such as liquid paraffin), an emollient (such as soft white paraffin wax) and consistency agents (such as hard paraffin wax, white beeswax and ceresin), whose percentage content can be regulated to obtain a product of suitable consistency with an adequate "drop point", so as to guarantee its compatibility with manufacturing requirements (machinability) and use requirements (spreadability) and maintenance of the desired product consistency under the established storage conditions.

To guarantee that a stable W/O emulsion is obtained, a suitable quantity of an appropriate liposoluble emulsifying agent with a mainly lipophilic nature (such as Arlacel 582) can be included.

To prevent oxidation of the lipophilic constituents, an antioxidant (such as butyl hydroxytoluene) and a synergising agent with a chelating action (such as disodium edetate) can be included in the composition.

To maintain the microbiological characteristics of the finished product during its shelf life, a suitable preservative system consisting of propyl para-hydroxybenzoate can be included in the composition.

The composition may also include hydrating agents (such as allantoin, all-rac-α-tocopheryl acetate) and flavouring agents (such as vanilla flavouring and sweet flavouring).

The present invention further relates to a process for the preparation of said pharmaceutical compositions in stick form containing acyclovir.

The process comprises the following stages:
a) preparing a water-in-oil (W/O) emulsion,
b) pouring the W/O emulsion into suitable moulds,
c) cooling to obtain a stick, preferably at a temperature of under 0°C.

The product thus obtained can then be inserted in a primary container.

The topical painkilling pharmaceutical compositions in the form of solid sticks, O/W emulsions and vaginal pessaries containing acyclovir and sodium hyaluronate can be used for the prevention and/or treatment of the pain associated with *Herpes simplex labialis* and *genitalis* infections.

It has now surprisingly been found that the use of the pharmaceutical compositions according to the present invention produces a fast, significant pain reduction, as demonstrated by the clinical findings reported below.

Pain caused by *H. simplex labialis* or *genitalis* is significant right from the outset of the disease, when the virus is in its most active form. The pain generally declines spontaneously within 5-7 days if the *H. labialis* blister does not burst; however, in the very frequent event that the blister bursts, the pain continues until re-epithelialisation, because the burst blister is a lesion, characterised by scab formation. In the case of *H. genitalis* infections, the pain lasts much longer, partly due to the site of infection. Once again, bursting of the blisters is particularly dangerous, because it can give rise to bacterial and/or fungal infections.

It has now surprisingly been found that sodium hyaluronate with an average MW of 200 kDa, acts quickly and effectively to prevent and counteract the pain caused by the herpes blister as soon as it appears.

The painkilling effect is presumably due to the specific bond of the hyaluronic acid used in the present invention with pain-mediating receptors (nociceptors) which respond to dynorphin.

None of the acyclovir-based pharmaceutical compositions known to the prior art are able to perform this painkilling action, yet pain is strongly disabling for patients suffering from the viral infection, especially when the virus is manifested by immunodepressed patients, in whom the disease can persist for long periods.

Their totally unexpected painkilling action makes the pharmaceutical compositions disclosed here far more advantageous than the known compositions.

The advantages deriving from use of the stick are:
- extremely easy application without using the hands, thus reducing the risk of potential microbial contamination;
- transparency of the product at the site of application, thus increasing its acceptability to users;
- greater persistence of the active ingredient *in situ;*
- improved re-epithelialisation due to the presence of sodium hyaluronate.

As regards the cream, one of its advantageous characteristics is the longer residence time of the antiviral active ingredient *in situ,* its better spreadability and better re-epithelialising activity due to the presence of sodium hyaluronate.

Vaginal pessaries represent an innovative therapeutic approach to the treatment of HSV-2 infection, combining the known pharmacological properties of acyclovir with the painkilling activity of sodium hyaluronate. The presence of sodium hyaluronate improves the residence time *in situ* of the active ingredient, which is absorbed constantly and continuously by the vaginal mucosa, supplied by numerous blood vessels, thus ensuring a constant concentration of the active ingredient *in situ.*

According to a preferred aspect of the present invention, the topical painkilling pharmaceutical compositions according to the invention may be:
- a water-in-oil emulsion (W/O emulsion) in solid stick form containing 5% w/w acyclovir and sodium hyaluronate with an average MW of 200 kDa at the concentration of 0.2% w/w, for use in the prevention and/or treatment of the pain associated with *H. simplex labialis* infections;
- a water-in-oil emulsion (W/O emulsion) in cream form containing 5% w/w acyclovir and sodium hyaluronate with an average MW of 200 kDa at the concentration of 0.2% w/w, for use in the prevention and/or treatment of the pain associated with *H. simplex labialis* or *genitalis* infections;
- vaginal pessaries containing acyclovir at a concentration of between 3 and 6%, preferably 5% w/w, and sodium hyaluronate with an average MW of 200 kDa at the concentration of 0.2% w/w, for use in the prevention and/or treatment of the pain associated with *H. simplex genitalis* infections.

The painkilling pharmaceutical compositions according to the present invention are useful in the prevention and/or treatment of pain associated with *Herpes simplex labialis* or *Herpes simplex genitalis* infections.

The examples given below further illustrate the invention.

### Examples

### Example 1

### Qualitative/quantitative composition of 100 g of solid stick according to the present invention

| **Ingredients** | **Quantity (g)** |
|---|---|
| **Active constituents:** | |
| Acyclovir | 5.00 |
| Sodium hyaluronate, mean MW 200 Kda | 0.20 |

| **Excipients:** | |
|---|---|
| Liquid paraffin | 24.52 |
| Soft white paraffin wax | 31.42 |
| Hard paraffin wax | 14.43 |
| White beeswax | 9.60 |
| Ceresin | 2.82 |
| Arlacel 582 | 9.58 |
| Allantoin | 0.11 |
| all-rac-α-tocopheryl acetate | 0.11 |
| Propyl para-hydroxybenzoate | 0.04 |
| Butyl hydroxytoluene | 0.04 |
| Purified water | 1.92 |
| Disodium edetate | 0.11 |
| Vanilla flavouring | 0.05 |
| Sweet flavouring | 0.05 |

### Example 2

### Scheme of process for the preparation of a composition described in Example 1

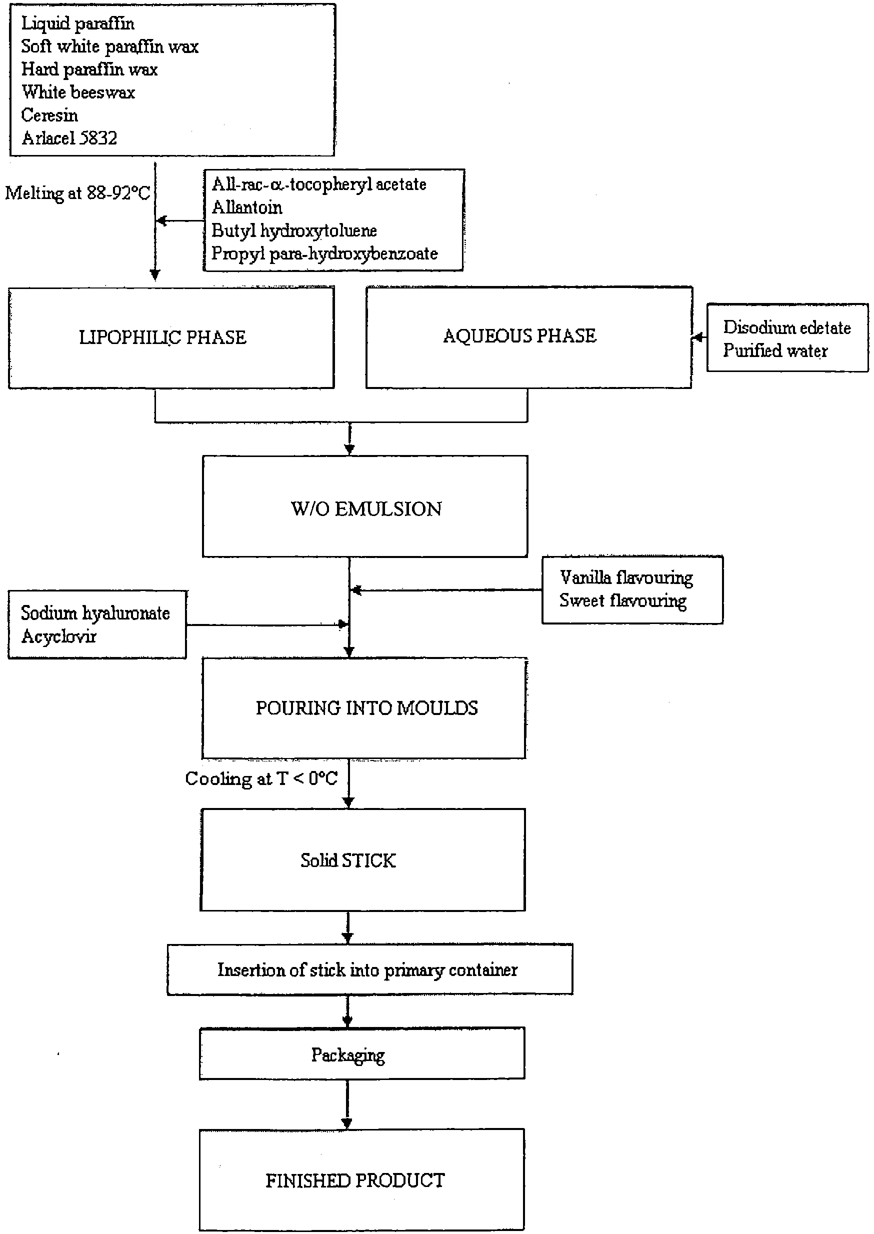

### Example 3

### Comparative study of the release and leachability properties of a solid stick according to the present invention containing 5% w/w acyclovir and a Zovirax^{®} cream containing 5% w/w acyclovir

The release and leachability measurements were taken simultaneously to evaluate the quantity of active ingredient available for release when the composition undergoes the leaching action of the physiological secretions (is "washed away"). This measurement was performed with a modified Franz diffusion cell.

The system, consisting of an upper "donor" compartment and a lower "receptor" compartment, was thermostated with an external jacket at the temperature of 32°C. The donor compartment has an air vent at the tip to allow air to exit during the compartment filling stage, and two side pipes to allow the entry and exit of the buffer (washability).

A phosphate buffer at pH 6.4 (USP29) was used as receiving phase to mimic the buccal environment, and as fluid that simulates saliva (with a flow rate of 0.5 ml/min at 32°C) in the donor compartment. The buffer was degassed before use and stirred during the measurements at a constant speed with a magnetic anchor in the receptor compartment. The compartments were separated by a dialysis membrane with a 12-14 kDa cut-off.

100 mg of the compositions tested (solid stick with 5% w/w acyclovir and sodium ialuronato according to the present invention and Zovirax cream with 5% w/w acyclovir) was applied to filter paper subsequently placed on the dialysis membrane. The donor compartment was secured to the receptor compartment with a clip.

To evaluate the quantity of active ingredient released by each composition, 500 µl of receptor phase was taken up at predetermined intervals for a total duration of 5 h.

To evaluate the quantity of active ingredient "washed away" by each composition, 1 ml of reflux buffer collected from the donor compartment was taken up at predetermined intervals for a total duration of 5 h. The volume taken up was replaced with fresh buffer each time. The active constituent was assayed by the HPLC method.

An apparatus (Perkin Elmer, I) fitted with a pump (binary pump, series 200), a UV/VIS detector (series 200) and an autosampler (series 200) was used for this purpose. The analysis method involved the use of a Supelco LC-18-DB column (5 µl, 250 x 4.6 mm) and a mobile phase, 97% (v/v) of which consisted of an 0.01 M solution of KH₂PO₄ and 3% (v/v) of acetonitrile. The process was conducted at ambient temperature with a flow rate of 1 ml/min and a capacity loop of 20 µl.

The data reported in figure 1 show that the active constituent formulated in stick form according to the present invention is much less leachable than the active constituent formulated in cream form (Zovirax^{®}).

The data reported in figure 2 show that the active constituent is released more slowly over time, thus ensuring a prolonged effect, when formulated in stick form according to the present invention than when formulated as a cream (Zovirax^{®}).

Figure 3 shows the peak release after 3 hours of approx. 0.3% of active ingredient in stick form according to the present invention, while figure 4 demonstrates that the release of the active ingredient in stick form according to the present invention remains stable over time.

### Example 4

### Comparative study of the painkilling activity of a solid stick according to the present invention compared with a solid stick with similar excipients containing 5% acyclovir

To assess the effect of sodium hyaluronate on pain caused by *H*. *simplex labialis,* it was tested on patients suffering from recurrent infections, focusing on two parameters of crucial importance:
- the pain symptoms notoriously associated with *H. simplex labialis* infections;
- healing of the lesion in terms of re-epithelialisation. In this respect it is essential to remember that the herpes blister does not always burst, leading to an ulcerative lesion; re-epithelialisation data were therefore only analysed for the patients in each group who manifested this event during the treatment.

### Trial protocol

Patients: 30 patients (12 male, 18 female, aged 21 to 34 years) were divided into two groups:
- Group A: 15 patients (6 m., 9 f.) treated with a stick prepared as described in Examples 1 and 2 of the present invention, therefore containing 5% acyclovir and 0.2% sodium hyaluronate, applied 5 times a day at intervals of approx. 4 hours;
- Group B: 15 patients (6 m., 9 f.) treated with a stick prepared as described in Examples 1 and 2 of the present invention, therefore containing 5% acyclovir but not containing sodium hyaluronate, applied 5 times a day at intervals of approx. 4 hours.

### Pain assessment

At the end of the day, each patient was asked to indicate in a questionnaire the level of pain present compared with that perceived at the start of the day, before the product was applied.
Subjective assessment scale: 0 = no pain; 5 = maximum pain,
Period considered: 5 days. After this period, the pain generally tends to decline spontaneously.

### Assessment of healing in terms of re-epithelialisation

Patients whose blisters burst and ulcerated were asked to record the re-epithelialisation time of the lesion formed.

Observation time: 10 days from the time when the blister burst.

### Analysis of results

### Pain assessment

Group A (Table 1) reported an immediate, clear pain reduction from the time of administration on the first day, and this reduction continued in the subsequent days, until day 5 of the assessment, when all patients reported total absence of pain.

The patients in Group B also reported a modest pain reduction, very probably due to the soothing effect of the excipients of the stick which reduces discomfort, and naturally to the pharmacological effect of acyclovir, which gradually deactivates the virus. However, total absence of pain was never reported, even close to day 5, when pain has physiologically declined compared with the starting condition.

**Table 1**

| **Mean value of pain perception** | | | | | |
|---|---|---|---|---|---|
| Group | Day **1** | Day **2** | Day **3** | Day **4** | Day **5** |
| **A**: n=15 | 2.4 | 1.9 | 0.5 | 0.0 | 0.0 |
| **B**: n=15 | 5.0 | 4.3 | 3.9 | 3.1 | 2.3 |
| **Scale:** 0 = no pain; 5 = maximum pain | | | | | |

As will be seen, the solid stick prepared as described in Examples 1 and 2 of the present invention, containing 5% acyclovir and 0.2% sodium hyaluronate, which was used by Group A, massively counteracts the pain deriving from the herpes lesion. As the only difference with the product used by Group B was the presence of sodium hyaluronate, the unexpected analgesic effect must be attributed to that substance.

### Assessment of healing

Herpes blisters burst in 6 patients (3m., 3f.) in Group A and 11 patients (5m., 6f.) in Group B.

Table 2 shows the progress of re-epithelialisation, calculated according to the protocol.

**Table 2**

| **Mean re-epithelialisation value** | | | | | | | | | | |
|---|---|---|---|---|---|---|---|---|---|---|
| Group | Day 1 | Day 2 | Day 3 | Day 4 | Day 5 | Day 6 | Day 7 | Day 8 | Day 9 | Day 10 |
| **A**: n=6 | 0 | 1.1 | 2 | 2.9 | 3.9 | 4.0 | 4.6 | 5.0 | 5.0 | 5.0 |
| **B**: n=11 | 0 | 0.3 | 1.0 | 1.9 | 2.5 | 2.9 | 3.2 | 3.3 | 3.7 | 3.9 |
| **Scale:** 0= no re-epithelialisation: 5= complete re-epithelialisation | | | | | | | | | | |

In Group A, re-epithelialisation clearly proceeded rapidly; it amounted to over 50% by day 5, and was complete for all patients by day 10 of the observation. In Group B, re-epithelialisation was much slower, and was not complete for all patients during the observation period.

Moreover, herpes blisters burst in fewer patients in Group A than Group B; this suggests that sodium hyaluronate exercises a protective effect on the labial mucosa, which is strengthened and made more elastic, and therefore less subject to ulcerative lesions.

The present invention therefore relates to a water-in-oil emulsion (W/O emulsion) in solid stick form which acts in various ways in the treatment of *H. simplex* lesions, as demonstrated:
- it increases the persistence of the active ingredient *in situ,* as it is less liable to leach;
- it causes slow release of the active ingredient;
- it leads to complete re-epithelialisation of the lesions in 10 days;
- it greatly reduces the pain associated with the disease.

### Example 5

### Qualitative/quantitative composition and preparation of 100 g of hydrophilic cream (O/W emulsion) containing 5% w/w acyclovir and 0.2% w/w sodium hyaluronate

| **Ingredients** | **Quantity (g)** |
|---|---|
| **Active ingredients** | |
| Acyclovir | 5.00 |
| Sodium hyaluronate (mean MW 200 Kda) | 0.20 |

| **Excipients:** | |
|---|---|
| PEG 400 monostearate | 10.0 |
| Decyl oleate | 5.0 |
| Emulsifying wax | 2.0 |
| Glycerol | 1.5 |
| 70% sorbitol solution | 1.5 |
| Methyl paraben | 0.2 |
| Propyl paraben | 0.02 |
| Sodium dehydroacetate | 0.2 |
| Flavouring | 0.05 |
| Purified water | q.s. for 100 |

### Preparation:

Load emulsifying wax, decyl oleate and PEG 400 monostearate into a turboemulsifier and heat at 80°C under stirring until a homogenous molten oily mass is obtained. Load the quantity of purified water into a dissolver, heat to 80°C and solubilise methyl paraben and propyl paraben. Add sodium hyaluronate, glycerol and sorbitol in solution, and finally sodium dehydroacetate, to this solution under stirring, until dissolution. Filter the said aqueous phase through an 0.45 micron membrane, transfer it to the turboemulsifier containing the oily phase, and emulsify. Cool the emulsion to 30°C, add acyclovir and homogenise. Cool the preparation to ambient temperature, and homogenise the fragrance in it.

### Example 6

### Qualitative/quantitative composition and preparation of 100 g of vaginal pessaries containing 5% w/w acyclovir and 0.2% w/w sodium hyaluronate

| **Ingredients** | **Quantity (g)** |
|---|---|
| **Active ingredients** | |
| Acyclovir | 5.00 |
| Sodium hyaluronate (mean MW 200 Kda) | 0.20 |

| **Excipients:** | |
|---|---|
| Glycerol | 61 |
| Gelatin | 17 |
| Purified water | q.s. for 100 |

### Preparation:

Solubilise sodium hyaluronate in water at 80°C; disperse the gelatin in the solution, finally adding the glycerol pre-heated to 80°C. Then add acyclovir under stirring and homogenise. Pour the mass into the moulds (1 pessary=3 g) and cool to ambient temperature.

## Claims

1. Topical painkilling pharmaceutical compositions in the form of a solid stick, O/W emulsion or vaginal pessaries comprising acyclovir at a concentration of between 2 and 8% w/w and sodium hyaluronate having an average molecular weight ranging from 160 to 200 kDa at a concentration of between 0.1 and 1% w/w for use in the prevention and/or treatment of the pain associated with *Herpes simplex labialis* and *genitalis* infections.

2. Pharmaceutical compositions for use as claimed in claim 1, wherein the acyclovir is in water-solvated form.

3. Pharmaceutical compositions for use as claimed in claim 1 or 2, wherein the acyclovir is present in a concentration of between 3 and 6% w/w, preferably 5% w/w.

4. Pharmaceutical compositions for use as claimed in claims 1-3, wherein sodium hyaluronate is present at the concentration of 0.2% w/w.

5. Pharmaceutical compositions for use as claimed in claims 1-4 in solid stick form containing 5% w/w acyclovir and sodium hyaluronate having an average MW of between 160 and 200 kDa at a concentration of 0.2% w/w for use in the prevention and/or treatment of the pain associated with *Herpes simplex labialis* infections.

6. Pharmaceutical compositions for use as claimed in claims 1-4 in the form of an oil-in-water emulsion containing 5% w/w acyclovir and sodium hyaluronate having an average MW of between 160 and 200 kDa at a concentration of 0.2% w/w for use in the prevention and/or treatment of the pain associated with *Herpes simplex labialis* or *genitalis* infections.

7. Pharmaceutical compositions for use as claimed in claims 1-4 in the form of vaginal pessaries containing acyclovir at a concentration of between 3 and 6% w/w, preferably 5% w/w, and sodium hyaluronate with an average MW of between 160 and 200 kDa at the concentration of 0.2% w/w for use in the prevention and/or treatment of pain associated with *Herpes simplex genitalis* infections.

8. Pharmaceutical compositions for use as claimed in claims 1-7 for use in the prevention and/or treatment of pain associated with *Herpes simplex labialis* or *genitalis* infections in immunodepressed patients.

## Patentansprüche

1. Topische, schmerzstillende, pharmazeutische Zusammensetzungen in Form eines festen Stiftes, einer Ö/W-Emulsion oder Scheidenpessaren, welche Acyclovir in einer Konzentration zwischen 2 und 8% w/w und Natriumhyaluronat mit einer mittleren Molekülmasse von 160 bis 200 kDa in einer Konzentration zwischen 0,1 und 1% w/w, zur Verwendung bei der Prävention und/oder Behandlung von Schmerzen, die mit Infektionen mit *Herpes simplex labialis* und *genitalis* assoziiert sind, umfasst.

2. Pharmazeutische Zusammensetzungen zur Verwendung gemäß Anspruch 1, wobei Acyclovir in wassersolvatisierter Form vorliegt.

3. Pharmazeutische Zusammensetzungen zur Verwendung gemäß Anspruch 1 oder 2, wobei Acyclovir in einer Konzentration zwischen 3 und 6% w/w, vorzugsweise von 5% w/w vorliegt.

4. Pharmazeutische Zusammensetzungen zur Verwendung gemäß Anspruch 1-3, wobei Natriumhyaluronat in einer Konzentration von 0,2% w/w vorliegt.

5. Pharmazeutische Zusammensetzungen zur Verwendung gemäß Anspruch 1-4 in Form eines festen Stifts, welcher 5% w/w Acyclovir und Natriumhyaluronat mit einer mittleren M von 160 bis 200 kDa in einer Konzentration von 0,2% w/w zur Verwendung bei der Prävention und/oder Behandlung von Schmerzen, die mit Infektionen mit *Herpes simplex labialis* assoziiert sind, enthält.

6. Pharmazeutische Zusammensetzungen zur Verwendung gemäß Anspruch 1-4 in Form einer Öl in Wasser Emulsion, welche 5% w/w Acyclovir und Natriumhyaluronat mit einer mittleren M von 160 bis 200 kDa in einer Konzentration von 0,2% w/w zur Verwendung bei der Prävention und/oder Behandlung von Schmerzen, die mit Infektionen mit *Herpes simplex labialis* oder *genitalis* assoziiert sind, enthält.

7. Pharmazeutische Zusammensetzungen zur Verwendung gemäß Anspruch 1-4 in Form von Scheidenpessaren, welche Acyclovir in einer Konzentration zwischen 3 und 6% w/w, vorzugsweise von 5% w/w und Natriumhyaluronat mit einer mittleren M von 160 bis 200 kDa in der Konzentration von 0,2% w/w zur Verwendung bei der Prävention und/oder Behandlung von Schmerzen, die mit Infektionen mit *Herpes simplex genitalis* assoziiert sind, enthält.

8. Pharmazeutische Zusammensetzungen zur Verwendung gemäß Anspruch 1-7 zur Verwendung bei der Prävention und/oder Behandlung von Schmerzen, die mit Infektionen mit *Herpes simplex labialis* oder *genitalis* assoziiert sind, bei immunsupprimierten Patienten.

## Revendications

1. Compositions pharmaceutiques antalgiques topiques sous forme de bâtonnet solide, d'émulsion huile-dans-eau ou de pessaire comprenant de l'acyclovir à une concentration comprise entre 2 et 8 % p/p et du hyaluronate de sodium ayant une masse moléculaire moyenne allant de 160 à 200 kDa à une concentration comprise entre 0,1 et 1 % p/p pour utilisation dans la prévention et/ou le traitement de la douleur associée aux infections à *Herpes simplex labialis* et *genitalis.*

2. Compositions pharmaceutiques pour utilisation selon la revendication 1, dans lesquelles l'acyclovir est sous une forme solvatée par l'eau.

3. Compositions pharmaceutiques pour utilisation selon la revendication 1 ou 2, dans lesquelles l'acyclovir est présent à une concentration comprise entre 3 et 6 % p/p, de préférence 5 % p/p.

4. Compositions pharmaceutiques pour utilisation selon les revendications 1 à 3, dans lesquelles le hyaluronate de sodium est présent à la concentration de 0,2 % p/p.

5. Compositions pharmaceutiques pour utilisation selon les revendications 1 à 4 sous forme de bâtonnet solide contenant 5 % p/p d'acyclovir et du hyaluronate de sodium ayant une masse moléculaire moyenne comprise entre 160 et 200 kDa à une concentration de 0,2 % p/p pour utilisation dans la prévention et/ou le traitement de la douleur associée aux infections à *Herpes simplex labialis.*

6. Compositions pharmaceutiques pour utilisation selon les revendications 1 à 4 sous forme d'émulsion huile-dans-eau contenant 5 % p/p d'acyclovir et du hyaluronate de sodium ayant une masse moléculaire moyenne comprise entre 160 et 200 kDa à une concentration de 0,2 % p/p pour utilisation dans la prévention et/ou le traitement de la douleur associée aux infections à *Herpes simplex labialis* ou *genitalis.*

7. Compositions pharmaceutiques pour utilisation selon les revendications 1 à 4 sous forme de pessaire vaginal contenant de l'acyclovir à une concentration comprise entre 3 et 6 % p/p, de préférence 5 % p/p, et du hyaluronate de sodium ayant une masse moléculaire moyenne comprise entre 160 et 200 kDa à la concentration de 0,2 % p/p pour utilisation dans la prévention et/ou le traitement de la douleur associée aux infections à *Herpes simplex genitalis.*

8. Compositions pharmaceutiques pour utilisation selon les revendications 1 à 7, pour utilisation dans la prévention et/ou le traitement de la douleur associée aux infections à *Herpes simplex labialis* ou *genitalis* chez les patients immunodéprimés.
